# EUROPEAN PATENT SPECIFICATION

(11) **EP 2 694 167 B1**
(45) Date of publication and mention of the grant of the patent: **02.05.2018**
(21) Application number: 12764486.2
(22) Date of filing: 30.03.2012
(51) Int. Cl.: A45F 3/14, A63B 23/02, A63B 71/08, B65G 7/12, A45F 3/04, A61F 5/02, A63B 21/06, A63B 21/00, A63B 21/065

(54) **SELF-ACTIVATED POSTURAL COMPLIANCE LIFT-ASSISTANCE DEVICE**
SELBSTAKTIVIERTE HEBEHILFSVORRICHTUNG MIT HALTUNGSNACHGIEBIGKEIT
DISPOSITIF D'ASSISTANCE AU LEVAGE À CONFORMITÉ POSTURALE ET À ACTIVATION AUTOMATIQUE

(30) Priority: 01.04.2011 US 201161516277 P; 06.02.2012 US 201261595187 P
(43) Date of publication of application: 12.02.2014
(73) Proprietor: Strong Arm Technologies, Inc., Rochester, NY 14623 (US)
(72) Inventor: PETTERSON, Sean, Michael, Mount Sinai, NY 11766 (US); HILLERY, Justin, Lamont, Rochester, NY 14609 (US)
(74) Representative: Durán-Corretjer, S.L.P.
(86) International application number: PCT/US2012/031440
(87) International publication number: WO 2012/135613

(56) References cited:
- EP-A1- 1 459 648
- EP-A1- 2 116 994
- EP-B1- 0 146 575
- WO-A2-00/41589
- DE-A1- 19 800 727
- GB-A- 988 796
- US-A- 2 651 441
- US-A- 5 499 965
- US-A- 5 656 020
- US-A1- 2005 000 994
- US-A1- 2010 075 817
- US-A1- 2010 075 817
- US-A1- 2010 076 359
- US-B1- 6 766 532

## Description

### FIELD OF THE INVENTION

The present invention preferably relates to a self-activated postural compliance lift-assistance device that puts the wearer in an increasingly supported lifting posture, thereby providing a lift-assistance device that conforms with best ergonomic practices for lifting.

### BACKGROUND OF THE INVENTION

According to the U.S. Occupational Health and Safety ("OSHA") technical manual, "back disorders can develop gradually as a result of microtrauma brought about by repetitive activity over time or can be the product of a single traumatic event... acute back injuries can be the immediate result of improper lifting techniques and/or lifting loads that are too heavy for the back to support." See OSHA technical manual, Section VII, Chapter I, "Back Disorders and Injuries," available at osha.gov/dts/osta/otm/otm_vii/otm_vii_1.html#app_vii:1_2 ("OSHA Manual"). As the OSHA Manual then goes on to note, "although back injuries account for no work-related deaths, they ... are one of the leading causes of disability for people in their working years and afflict over 600,000 employees each year with a cost of about $50 billion annually in 1991 according to NIOSH ... [and] the frequency and economic impact of back injuries and disorders on the work force are expected to increase over the next several decades as the average age of the work force increases and medical costs go up."

Given the enormous health and economic consequences of lifting-related back injuries, there have been a large number of devices developed that purport to be useful for better lifting safety. See, e.g., the numerous examples of such devices within U.S. Classification Class/Subclass 602/19. However, in 1994 a "Back Belt Working Group" of the National Institute of Occupational Health and Safety ("NIOSH") reviewed commercially available lifting belts and concluded that such "back belts do not mitigate hazards to workers posed by repeated lifting, pushing, pulling, twisting, or bending" and that, in light of "insufficient data indicating that typical industrial back belts significantly reduce the biomechanical loading of the trunk during manual lifting," this working group concluded that 1) back belts are not recommended for preventing injuries; and, 2) back belts are not personal protective equipment ("PPE"). See NIOSH publication 94-122, available at cdc.gov/niosh/docs/94-122/ ("NIOSH1994"). See also NIOSH's 1996 summary of these results, NIOSH publication 94-127, October, 1996, available at cdc.gov/niosh/docs/94-127/ ("NIOSH1996"). US 2005/000994 A1 discloses a carrying assistance device comprising load transfer means comprising a pair of straps, postural compliance means formed as a plate, and coupling means for coupling the load transfer means to the postural compliance means.

In light of the above health and economic consequences of lifting-related back injuries and the lack of suitable devices for preventing such injuries, there is a great need for the development of better lift-assistance devices.

### SUMMARY OF THE INVENTION

The present invention relates to a self-activated postural compliance lift-assistance device that puts the wearer in an increasingly supported lifting posture, thereby providing a lift-assistance device that conforms with best ergonomic practices for lifting. The invention relates to a lift-assistance device comprising the features of claim 1, and to a method comprising the features of claim 12. Advantageous features are presented in the dependent claims.

### BRIEF DESCRIPTION OF THE DRAWINGS

The drawings provided in the present invention are provided solely to better illustrate particular embodiments of the present invention, and specifically do not provide an exhaustive or limiting set of embodiments of the present invention.
Figure 1 provides a schematic example of non-ergonomic lifting, i.e., lifting by keeping the legs straight/locked and bending at the waist with a hunched back.
Figure 2 provides a schematic example of ergonomic lifting, which involves keeping the weight as close to the body as possible, keeping the torso relatively erect to preserve the natural curvature of the spine, and using the leg muscles to do the lifting, e.g., by going from a squat to a standing position.
Figure 3 provides a schematic example of a simple device intended to put the user in an appropriate lifting posture. As long as the wearer keeps his/her back relatively erect, increasing loading on the two straps will pull the user further upright, that is, into the appropriate conformation for lifting.
Figure 4 shows that a device as simple as that shown in Figure 3 will not function appropriately because the user will naturally tend to hunch over, thereby worsening the wearer's posture and putting even greater loading on his/her spine
Figure 5 provides a schematic example of one non-limiting embodiment, which is not part of the present invention, directed to a device that has at least the additional functionality of either preventing hunching over or encouraging erect posture, or a combination of the two. The view in this figure is of the back of the torso.
Figure 6 provides a more generalized schematic example of an embodiment which is not part of the invention in which the load-transfer means **LTM** (e.g., straps **S1** and **S2)** transfers the load from the lifting point over the shoulders and down to the waist belt **W,** where the weight is then transferred via coupling means **C** to the postural compliance means **PCM,** which upon increased loading increasingly engages to ensure the appropriate lifting posture of a non-loaded curve of the spine and prevents/enforces non-hunching.
Figure 7 provides a schematic of an embodiment of the invention with the **LTM, PCM** and **C** means described above; in this embodiment there is a single coupling means **C** that rides in a vertical channel in slide **SL,** where motion of **C** vertically in the channel of slide SL results in the coupling of increased weight on the load-transfer means **LTM** to increasingly enforced postural compliance via tightening of the **PCM.**
Figure 8 provides a photograph of a prototype lifting vest of the embodiment of the present invention shown schematically in Figure 7.
Figure 9 provides an exploded view of the coupling means **C** in the channeled slider **SL** of the embodiment shown in Figure 7.
Figures 10-13 show various additional exemplary embodiments which are not part of the present invention.
Figure 14 provides a super-positioning of images obtained at various stages during the lifting process of a user wearing one embodiment of the present invention.
Figure 15 provides data on lumbar kinematics during weight-lifting without and with an exemplary lift-assistance device of the present invention. These data show that the lift-assistance device has a significant effect on reducing peak lumbar flexion during lifting, with the reduction seen for heavier loads reaching over 50%.
Figures 16-17 show various embodiments of the "load coupling means" of the present invention.
Figures 18-20 show one embodiment of the "load-activated grip-assisting glove" embodiment contemplated in the present invention.

### DETAILED DESCRIPTION OF THE INVENTION

Note that in the present invention, "a" or "an" are explicitly not limited to the singular form; instead, "a" and "an" are explicitly intended to be synonymous with "at least - but not limited to - one instance of" the term being referenced.

### Appropriate Lifting Posture/Sequence of Lifting Postures

The present invention is based on the recognition that lifting-related injuries can be significantly reduced by: 1) compliance with the appropriate sequence of postures during lifting; and, 2) mechanical distribution of weight across the body as determined by ergonomic studies. In order to implement 1) and 2) above the present invention is particularly directed to an unconventional device for insuring sequenced postural compliance and appropriate weight distribution, while also supplying a third critical factor of 3) a device design that is comfortably donned/removed and worn, in order to prevent user non-compliance, within 4) appropriate manufacturability parameters, e.g., durability and price.

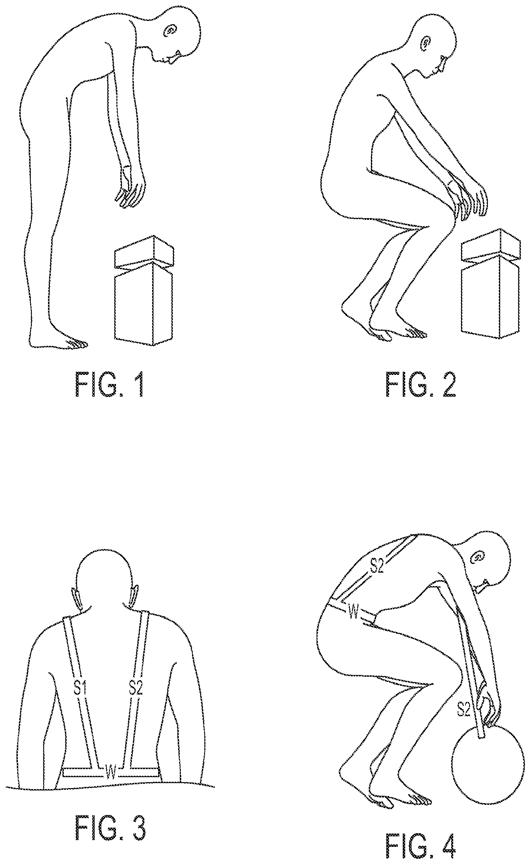

With regard to the first factor, the appropriate sequence of postures during lifting, a large number of ergonomic studies have established a standard sequence of postures for lifting. The Mayo Clinic, for example, lists a lifting sequence consisting of 6 steps: 1) start in a safe position; 2) maintain the natural curve in your lower back; 3) use your legs; 4) squatting instead of kneeling; 5) let your legs do the work; and, 6) avoid twisting. See mayoclinic.com/health/back-pain/LB00004_D. This sequence of steps captures the two basic principles of a) not lifting at the waist, and instead b) lifting with the back relatively erect, using the legs. Thus as shown in Figure 1, lifting by keeping the legs straight/locked and bending at the waist with a hunched back is non-ergonomic lifting, since lifting in this posture forces the spine to support both the weight of the upper body and the weight of the load being lifted and, worse, the distance of the load out from the center of the body in this posture enormously increases the strain acting on the spine, e.g., into the thousands of foot-pounds of torque. See, e.g., "Biomechanics of Safe Lifting, available at ergo.human.cornell.edu/DEA3250notes/lifting.html. See also, e.g., "Applications Manual for the Revised NIOSH Lifting Equation," 1994, NIOSH publication PB94-176930.

Instead, as shown in Figure 2, ergonomic lifting involves keeping the weight as close to the body as possible, keeping the torso relatively erect to preserve the natural curvature of the spine, and using the leg muscles to do the lifting, e.g., by going from a squat to a standing position. In this regard, it is worthwhile nothing that, according to NIOSH1996, "[i]t would appear that abdominal belts help restrict the range of motion during side to side bending and twisting. However, they do not have the same effect when the worker bends forward, as in many industrial lifting situations." Thus it would appear that current support belts generally have little if any effect on ensuring this correct lifting posture and, as a result, a worker wearing a conventional lifting belt is unlikely to adopt this posture - keeping the weight close to the body by minimizing bending from the waist (thereby keeping the torso upright and lowering the stresses on the spine) - or the coupled requirement for proper lifting of using the legs to lift, i.e., by going from a squat to an erect position during lifting.

### The Lift-Assistance Device of the Present Invention

In this regard, in one aspect the present invention is directed to ensuring that a wearer appropriately lifts loads 1) with the back in a series of positions that - as the loading increases - becomes increasingly constrained to be erect (i.e., a "self-activated" device), thereby ensuring that the user's spine experiences minimized loading during lifting and particularly when lifting the full load. Referring to Figure 3, a simple mechanical device for achieving this purpose and which is not part of the invention might consist of two straps (**S1** and **S2**) attached at a waist belt (**W**) and going over one or more - and preferably both - of the two shoulders down to the weight being lifted in front, with the distal (far) ends of these straps ending either at the hands or in hooks or other grips that directly contact the weight being lifted. As long as the wearer keeps his/her back relatively erect, increasing loading on the two straps will pull the user further upright, that is, into the appropriate conformation for lifting.

In reality, a device as simple as that shown in Figure 3 will not function appropriately because the user will naturally tend to hunch over, thereby worsening the wearer's posture and putting even greater loading on his/her spine, a situation that is shown in Figure 4. Note that the weight in this figure is shown as being a sphere; also, **S2** is shown as extending down directly to the weight, although this is only one embodiment of the present invention. **S1** is not shown in this figure, but will have a form similar to that of **S2.**

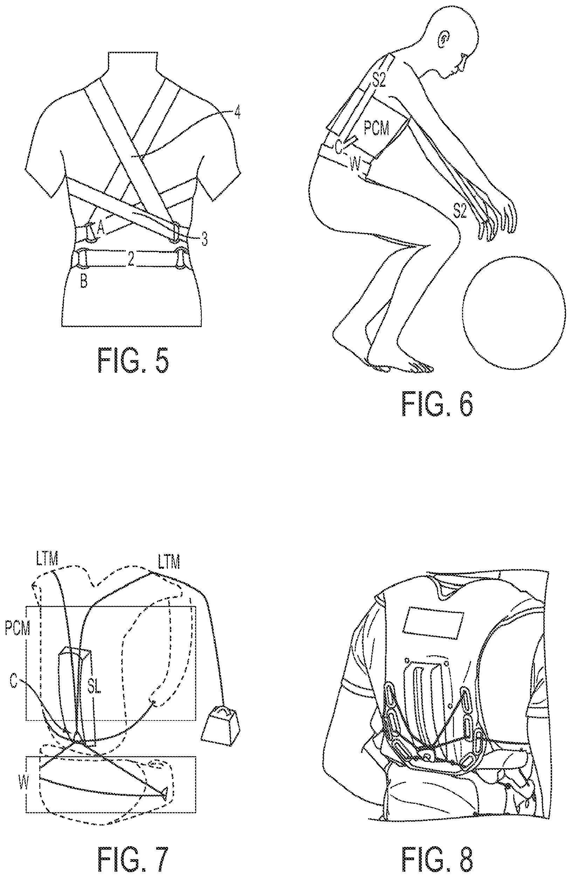

In order to prevent the situation depicted in, e.g., Figure 4, one embodiment is directed to a device that has at least the additional functionality of either preventing hunching over or encouraging erect posture, or a combination of the two. Figure 5 provides one embodiment of such a device, which is not part of the invention. Specifically, Figure 5 shows two shoulder straps **4** (for clarity, only the shoulder strap crossing the left shoulder is labeled in this schematic, but the designation refers to both straps) that in this embodiment criss-cross the shoulders and descend across the back to D-ring "coupling means" (**A**; although only the D-ring on the left side of the wearer's body is labeled in this figure, the designation also refers to the corresponding D-ring on the right side) or other (non-D-ring) forms of coupling means that allow the shoulder straps **4** to descend down to or near to the waist (in the embodiment of Figure 4 the D-ring coupling means **A** are fixed in position directly above the hip/waist belt **2;** in general the invention contemplates one or more attachment points on the torso, preferably the lower torso, and still more preferably in the region of the waist) and that, under loading, allow the continuation of these shoulder straps **3** to slide through these D-ring coupling means **A.** Since these continuation straps **3** continue on around the body where they are fixed (these ends are not shown in the figure), loading on straps **4** results in tensioning of straps **3** through sliding of the straps through the coupling means **A,** with the tensioning of straps **3** compressing the torso so as to support/alter the wearer's position to a more erect posture, thereby ensuring postural compliance that prevents the situation shown in Figure 4.

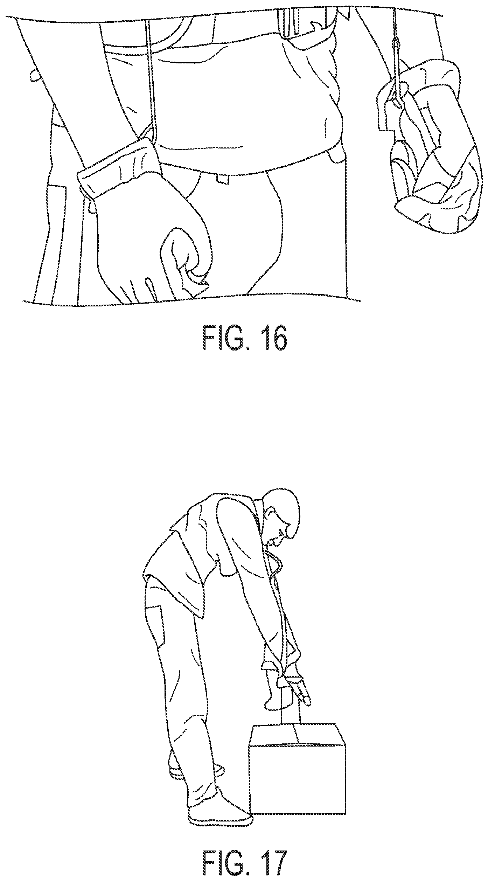

Figure 6 shows a more generalized schematic representation of this embodiment which is not part of the present invention, where the straps **S1** and **S2** (only **S2** is shown; **S1** is the mirror image of **S2,** in that it attaches in the region of the right hand and crosses the left shoulder in this criss-crossed **S1/S2** embodiment) ascend from an attached positioning at the "lifting point" in the region of the lower forearm/hands (e.g., by "lift coupling means" such as gloves, lifting hooks, wrist-straps, etc., as described in more detail below and in, e.g., Figures 16-17), over the shoulders (in either crossed or uncrossed conformations) and down across the back to the waist belt **W,** where the straps are connected via coupling means **C** (here on each side of the body) to the postural compliance means **PCM** of the apparatus. In this representation, the coupling means **C** for each strap **S1** and **S2** could be the D-ring coupling means of Figure 5, although other coupling means are contemplated (see below). The postural compliance means **PCM** could be, e.g., the straps 3 of Figure 5 that compress the torso upon loading of the straps, although this is only one of the embodiments contemplated for the postural compliance means **PCM.**

Thus in the embodiment depicted in Figure 6, there are three critical sections to the embodiment: 1) the load-transfer means **LTM,** e.g., straps **S1** and **S2** (although the term "LTM" encompasses fewer or more straps, non-strap means such as ropes or strings, etc.), which transfers the load weighting from the lifting point (hands, wrists, forearms, etc.) over the shoulders and down to the lower torso, typically the waist belt **W** (again, the invention most generally contemplates one or more attachment points on the torso, preferably the lower torso, and still more preferably in the waist region); 2) the postural compliance means **PCM,** which upon increased loading increasingly engages to ensure the appropriate lifting posture of a non-loaded curve of the spine and prevents/enforces non-hunching (i.e., prevents the inappropriate back position of Figure 4); and, 3) coupling means **C** (multiple coupling means are shown in this figure, but other numbers of such coupling means are contemplated, as in, e.g., Figure 7), which allows increased loading on the **LTM** such as **S1/S2** to be translated into increasing engagement of the **PCM** and, therefore, increasing postural compliance.

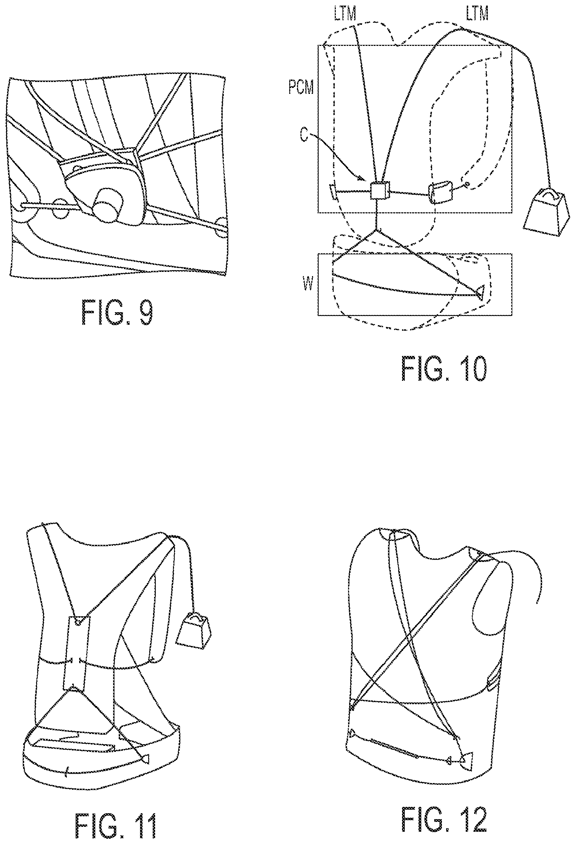

Figure 7 shows an embodiment of the invention with the **LTM, PCM** and **C** means described above. In this embodiment there is a single coupling means **C** configured to slide up and down along a channeled slider **SL** that is placed approximately mid-torso over the spine; as this coupling means **C** ascends the channel as a result of the downward motion of the load-transfer means **LTM** at their attachment points to the load (show in the figure as a rectangular weight with a handle attached to the "right" **LTM** (i.e., the **LTM** that descends to the wearer's right hand); the corresponding weight on the left **LTM** is not shown), the postural compliance means **PCM** compresses the torso - in this embodiment via the drawing in of the shoulder straps - to the appropriate lifting posture. Figure 8 provides a photograph of a prototype of the embodiment of Figure 7; Figure 9 provides an exploded view of the coupling means **C** in the channeled slider **SL** of this embodiment. Figures 10-13 show additional exemplary embodiments which are not part of the present invention, all of which embody the same basic principle of coupling between lifting and the **PCM** via a single coupling means or multiple coupling means.

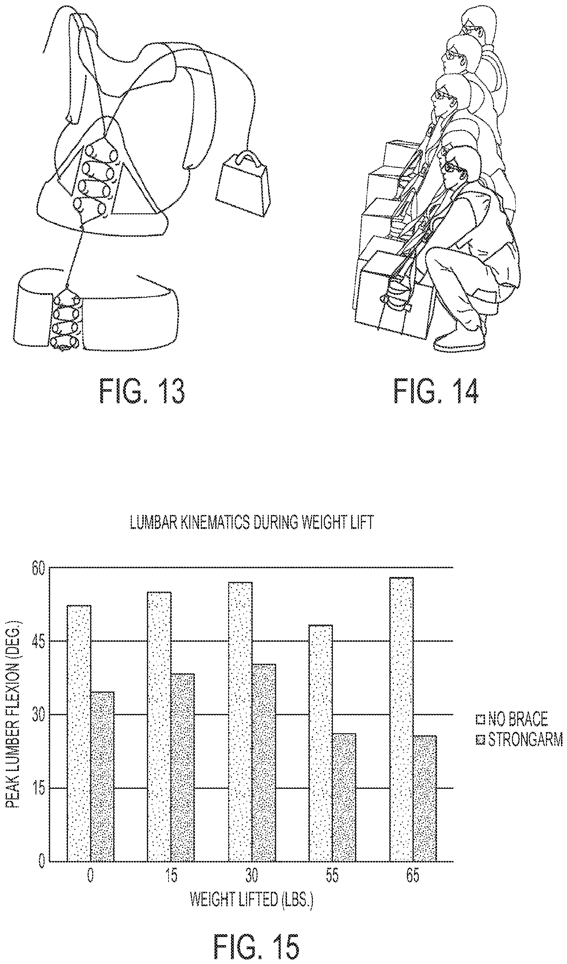

As discussed above, as loading on the load-transfer means **LTM** increases, so too does the postural compliance exerted by the postural compliance means **PCM,** with the coupling between the two obtained by at least one coupling means **C.** Figure 14 provides a super-positioning of images obtained at various stages during the lifting process of a user wearing one embodiment of the present invention; as this figure shows, the back remains in appropriate posture throughout lifting, with a gradated change in posture during lifting to preserve the appropriate posture.

With regard to the change in the postural compliance enforced by the **PCM,** this change can be linear, or it can be non-linear. Thus for example the **PCM** may be gradually engaged via increased tensioning of straps as in the embodiment of, e.g., Figure 5; alternatively the **PCM** may be designed so that the **PCM** engages as an full-off or full-on process when sufficient lifting weight in the **LTM.**

### Measured Ergonomic Effects of the Lift-Assistance Device

As already discussed, in 1994 a "Back Belt Working Group" of the National Institute of Occupational Health and Safety ("NIOSH") reviewed commercially available lifting belts and concluded that there were insufficient data to indicate "that typical industrial back belts significantly reduce the biomechanical loading of the trunk during manual lifting." For the present lift-assistance device such data have been obtained; as the exemplary data in Figure 15 show, this device has a significant effect on reducing peak lumbar flexion during lifting, with the reduction seen for heavier loads reaching over 50%. Thus these data can be used to define one embodiment of the invention, where the sequence of "appropriate lifting postures" that occur with engagement of the **PCM** are such as to reduce peak lumbar flexion at any particular weight at least to the extent shown in Figure 15.

### "Lifting Point" Embodiments

As already discussed, **LTM** generically refers to the means of the present invention for transferring the load weighting from the "lifting point" (hands, wrists, forearms, etc.) over the shoulders and down to the lower torso. Further with regard to specific terminology, at the lifting point on each arm different "lift coupling means" ("**LCM**") may be used to couple the **LTM** ends such as string or strap or wire ends to a hook, glove or other lift coupling means that serves to transfer the weight of the object(s) to be lifted directly to the **LTM.** Two examples of such **LCM** are shown in Figures 16 and 17; specifically, Figure 16 shows **LCM** in the form of gloves that attach to the **LTM** via a hook, loop, grommet, etc., while Figure 17 shows attachment to lifting hooks instead of gloves.

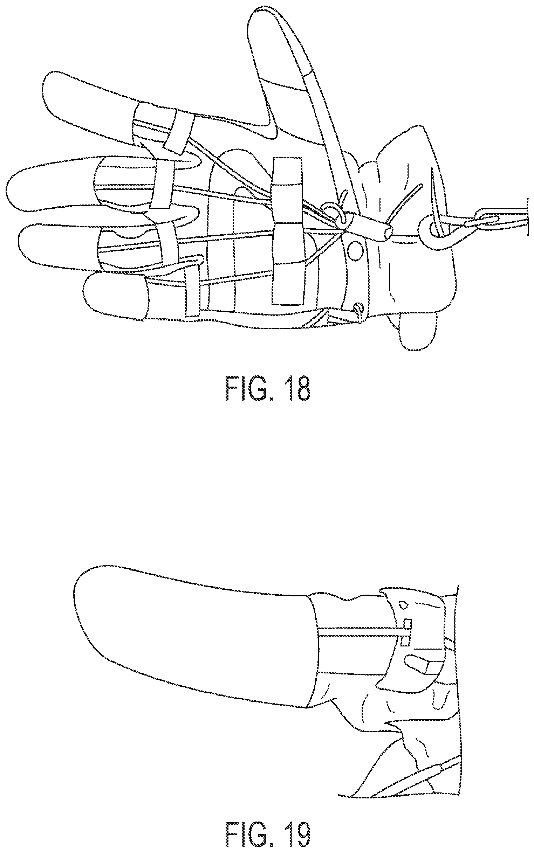

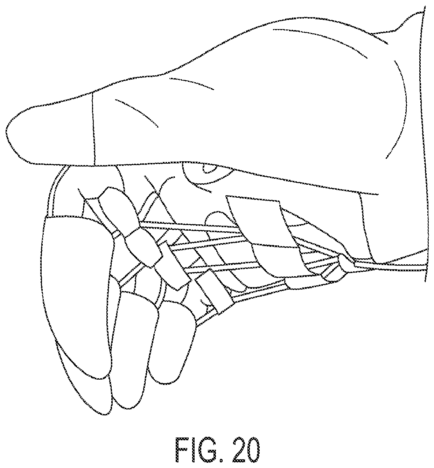

With regard to the various **LCM** embodiments of the present invention, another non-limiting embodiment of an **LCM** is the "load-activated grip-assisting glove" embodiment provided in Figures 18-20. In this **LCM** embodiment, a specially-designed glove is intended to increase users grip strength when picking up a load. The device has linear members that run from the fingertips and end at an attachment point at the wrist. The device is worn like a glove and it attaches to the wearer's arm or body, in this case the postural conformance device via the attachment point. When the wearer lifts a load, the forces of that load force the hand into a grip by pulling the string taught therefore curling the fingers. The pieces on the mid finger and the hard finger tips force the fingers to curl in a specific orientation, e.g. in one preferred but non-limiting embodiment a hook-like shape (e.g., the conformation of the device in Figure 20 versus Figure 18).

The present invention contemplates various forms of **LCM,** each of which may be particularly suited to the needs of a worker in a different work environment. Thus for example hook **LCM** may be particularly appropriate for a worker lifting small boxes, whereas glove **LCM** may be more appropriate for workers lifting a variety of oddly sized, hard to grip objects. Other non-limiting examples of **LCM** include, for example, mechanical or electrical grippers, engagement posts, etc.

Finally, Applicants note that Figure 17 shows that although the **LCM** of the present invention can be in the form of free-standing straps, wires, strings, etc., that are not constrained to run along at least some part of the upper and lower arm, in some embodiments (e.g., that of Figure 17) the **LCM** are constrained to run along at least some length of the arm. Such an embodiment is preferred in a variety of workplace environments where fouling of the straps/wires/strings of the **LCM** would occur if these were free-standing.

### Sensor-Laden Lift-Assistance Device

In additional embodiments, the present invention is directed to a lift-assistance device or vest that includes feedback sensors to indicate directly to the user, or by telemetry to a telemetry-storage device or remote telemetry network various data on user lifting.

Thus for example, in one embodiment, the lift-assistance device of the invention includes "unsafe weight" mechanical sensors that trip to indicate to the user that a weight outside of safe-lifting parameters is being lifted. Thus in one non-limiting embodiment, each **LTM** may have installed in it a mechanical device that, upon sufficient weighting, elongates with a pronounced noise, or that, upon elongation, exposes a colored "weight exceeded" color, or some combination of these indicators, to indicate to the user that the weight being lifted is unsafe for that user. Note that this "unsafe weight" may be a fixed weight, or it may be a weight that varies as a function of time-of-day, amount of weight already lifted by the user over the course of the day or in the last time period, some combination of the above, etc. Although the present invention contemplates unsafe weight sensors as typically being mechanical in nature, sensors that similarly signal unsafe weight using electrical means are also explicitly contemplated.

In other embodiments, the on-vest/on-body sensor(s) may transmit load/elongation data from multiple points on-vest/on-body, where such transmission is either wired or, preferably, wireless (e.g., by Bluetooth) to an on-body recording device, an on-body indicator/retransmission device (e.g., a smartphone application), an off-body receiver network, or some combination of the above. Intermittently- or continuously-transmitted data of this sort may be collected for a variety of purposes, including a) feedback to the vest-wearer regarding appropriate load lifting over the course of the day (e.g, as estimated by one or more algorithms regarding user capacity for additional lifting given previous lifts, time of day, state of body, etc.); b) data collection regarding lifting for correlation with injuries (i.e., to collect data for the development of safer-lifting algorithms); c) data collection for employer implementation of optimized worker lifting (e.g., real-time redistribution of workers based on metrics of each worker's approach to maximum lifting per day, per hour, etc., so that efficiency is maximized while likelihood of worker injuries is minimized by ensuring workers are not being overtasked for lifting). The present invention includes not just the hardware required for such implementations, but also the associated software, including software for a) data acquisition and processing; b) data-mining to extract safe lifting algorithm(s); data processing to coordinate workers, with additional software layers to ensure masking of data or other individual privacy layers to ensure protection of employees from inappropriate employer monitoring, etc.

The scope of the invention is defined by the claims.

## Claims

1. A lift-assistance device comprising:
i) a load transfer means (LTM), comprising a pair of straps (S1, S2), each strap (S1, S2) configured to extend from a lifting point over a respective shoulder of the wearer and down to one or more points on the torso;
ii) a postural compliance means (PCM), for either passively or actively enforcing the appropriate back posture or/and sequence of back postures; and,
iii) a coupling means (C), for coupling the pair of straps (S1, S2) of the load-transfer means (LTM) to respective points of the postural compliance means (PCM) such that the pair of straps (S1, S2) exert a force on the postural compliance means (PCM) in a direction that is not parallel to a direction of extension of the wearer's spine, the coupling means (C) being configured to be positioned adjacent the wearer's spine, each strap (S1, S2) of the load transfer means (LTM) coupled to the respective point of the postural compliance means (PCM) via the coupling means (C), **characterized in that** a channeled slider is configured to be positioned adjacent the wearer's spine on the postural compliance means (PCM), wherein the coupling means is slidable along a channel defined by the channeled slider (SL) and extending in the direction of extension of the wearer's spine.

2. The lift-assistance device of claim 1, wherein the appropriate back posture for each engagement level of the postural compliance means (PCM) is one that promotes maintenance of a natural curve of the user's back at that engagement level of the postural compliance means (PCM).

3. The lift-assistance device of claim 1, wherein the appropriate back posture for each engagement level of the postural compliance means (PCM) is one that reduces peak lumbar flexion at that engagement level of the postural compliance means (PCM).

4. The lift-assistance device of claim 1, further comprising a lift coupling means for each of the user's arms, wherein each lift coupling means transfers at least part of the weight of the load to be lifted to the load transfer means (LTM) for that arm.

5. The lift-assistance device of claim 4, where the lift coupling means is selected from the group consisting of gloves, hooks, grippers and gripping strips, and hook and loop fasteners.

6. The lift-assistance device of claim 4, where the lift coupling means is at least one glove comprising a plurality of linear members extending from fingertips of the glove to a central point adjacent a wrist portion of the glove, said central point adapted for coupling to the load transfer means (LTM).

7. The lift-assistance device of claim 1, wherein increasing engagement of the postural compliance means is linearly related to the weight supported by the load transfer means (LTM).

8. The lift-assistance device of claim 1, wherein increasing engagement of the postural compliance means (PCM) is non-linearly related to the weight supported by the load transfer means (LTM).

9. The lift-assistance device of claim 8, wherein increasing engagement of the postural compliance means (PCM) is a bi-state engagement from disengaged state to fully engaged state.

10. The lift-assistance device of claim 1, further comprising one or more sensors for assaying one or more of the load being lifted, loading at one or more points on the user's body or one or more indicators of strain on the user's body from lifting.

11. The lift-assistance device of claim 10, wherein the one or more sensors includes one or more unsafe-weight sensors.

12. A method for reducing lifting-related injuries comprising lifting while wearing a lift-assistance device comprising:
i) transferring a load weighting from a lifting point over shoulders of a user and down to one or more points on the user's torso via a load transfer means (LTM) that comprises a pair of straps (S1, S2) that extend from the lifting point over respective shoulders of the user and down to the one or more points on the user's torso;
ii) either passively or actively enforcing either one or both of an appropriate back posture and a sequence of back postures with a postural compliance means (PCM);
iii) coupling on the pair of straps (S1, S2) of the load transfer means (LTM) via a coupling means (C) such that the pair of straps (S1, S2) exert a force on the postural compliance means (PCM) in a direction that is not parallel to a direction of extension of the wearer's spine, the coupling means (C) being configured to be positioned adjacent the wearer's spine, each strap (S1, S2) of the load transfer means (LTM) coupled to the respective point of the postural compliance means (PCM) via the coupling means (C); and
iv) sliding the coupling means (C) along a channel extending in the direction of extension of the wearer's spine, the channel defined by a channeled slider (SL) positioned adjacent the wearer's spine on the postural compliance means (PCM).

## Patentansprüche

1. Hebeunterstützungsvorrichtung, umfassend:
i) ein Lastübertragungsmittel (LMT; für Englisch: load transfer means), umfassend ein Gurtpaar (S1, S2; für Englisch: strap), wobei jeder Gurt (S1, S2) so konfiguriert ist, dass er sich von einem Hebepunkt über eine entsprechende Schulter des Trägers und hinunter zu einem oder mehreren Punkten an dem Torso erstreckt;
ii) ein Haltungserfüllungsmittel (PCM; für Englisch: postural compliance means) zur entweder passiven oder aktiven Erzwingung der angemes-senen Rückenhaltung und/oder einer Abfolge von Rückenhaltungen; und
iii) ein Verbindungsmittel (C; für Englisch: coupling means), um das Gurtpaar (S1, S2) des Lastübertragungsmittels (LTM) mit entsprechende Punkten des Haltungserfüllungsmittels (PCM) zu verbinden, so dass das Gurtpaar (S1, S2) eine Kraft auf das Haltungserfüllungsmittel (PCM) in einer Richtung ausübt, die nicht zu einer Ausdehnungsrichtung von der Wirbelsäule des Trägers parallel ist, wobei das Verbindungsmittel (C) so konfiguriert ist, dass es benachbart zur Wirbelsäule des Trägers positioniert ist, wobei jeder Gurt (S1, S2) des Lastübertragungsmittels (LTM) mit dem entsprechenden Punkt des Haltungserfüllungsmittels (PCM) über das Verbindungsmittel (C) verbunden ist, **dadurch gekennzeichnet, dass** ein kannelierter Schieber so konfiguriert ist, dass er benachbart zur Wirbelsäule des Trägers an dem Haltungserfüllungsmittel (PCM) positionierbar ist, wobei das Verbindungsmittel entlang eines Kanals verschiebbar ist, der durch den kannelierten Schieber (SL; für Englisch: slider) definiert wird und sich in der Ausdehnungsrichtung von der Wirbelsäule des Trägers erstreckt.

2. Hebeunterstützungsvorrichtung nach Anspruch 1, wobei die angemessene Rückenhaltung für jedes Eingriffsniveau von dem Haltungserfüllungsmittel (PCM) eine ist, welche die Aufrechterhaltung von einer natürlichen Krümmung der Wirbelsäule von dem Verwender bei diesem Eingriffsniveau von der Haltungserfüllungsvorrichtung (LTM) fördert.

3. Hebeunterstützungsvorrichtung nach Anspruch 1, wobei die angemessene Rückenhaltung für jedes Eingriffsniveau des Haltungserfüllungsmittel (PCM) eine ist, welche die lumbale Spitzenflexion bei diesem Eingriffsniveau von der Haltungserfüllungsvorrichtung (PCM) verringert.

4. Hebeunterstützungsvorrichtung nach Anspruch 1, ferner umfassend ein Hebeverbindungsmittel für jeden Arm des Verwenders, wobei jedes Hebeverbindungsmittel mindestens einen Teil des Gewichts von der anzuhebenden Last auf das Lastübertragungsmittel (LTM) für diesen Arm überträgt.

5. Hebeunterstützungsvorrichtung nach Anspruch 4, wobei das Hebeverbindungsmittel aus der Gruppe ausgewählt ist, bestehend aus Handschuhen, Haken, Greifern und Griffleisten, und Klettverschlüssen.

6. Hebeunterstützungsvorrichtung nach Anspruch 4, wobei das Hebeverbindungsmittel mindestens ein Handschuh ist, der eine Vielzahl von linearen Elementen umfasst, die sich von den Fingerspritzen des Handschuhs zu einem zentralen Punkt benachbart zu einem Handgelenksteil des Handschuhs erstrecken, wobei der zentrale Punkt geeignet ist, um mit dem Lastübertragungsmittel (LTM) verbunden zu werden.

7. Hebeunterstützungsvorrichtung nach Anspruch 1, wobei ein zunehmender Eingriff des Haltungserfüllungsmittels in einem linearen Bezug zu dem Gewicht steht, dass durch das Lastübertragungsmittel (LTM) getragen wird.

8. Hebeunterstützungsvorrichtung nach Anspruch 1, wobei ein zunehmender Eingriff des Haltungserfüllungsmittels (PCM) in einem nicht-linearen Bezug zu dem Gewicht steht, dass durch das Lastübertragungsmittel (LTM) getragen wird.

9. Hebeunterstützungsvorrichtung nach Anspruch 8, wobei ein zunehmender Eingriff des Haltungserfüllungsmittels (PCM) ein Zwei-Zustand-Eingriff von einem nicht-eingegriffenen Zustand bis hin zu einem vollständig eingegriffenen Zustand ist.

10. Hebeunterstützungsvorrichtung nach Anspruch 1, ferner umfassend einen oder mehrere Sensoren, um eine oder mehrere der anzuhebenden Lasten, eine Belastung an einem oder mehreren Punkten an dem Körper des Verwenders oder einen oder mehrere Spannungsindikatoren an dem Körper des Verwenders durch das Heben zu prüfen.

11. Hebeunterstützungsvorrichtung nach Anspruch 10, wobei die ein oder mehreren Sensoren einen oder mehrere Sensoren für ein bedenkliches Gewicht umfassen.

12. Verfahren zur Verringerung hebebezogener Verletzungen, umfassend das Heben währenddessen eine Hebeunterstützungsvorrichtung getragen wird, umfassend:
i) Übertragen mittels eines Lastübertragungsmittels (LMT; für Englisch: load transfer means) von einem Lastgewicht von einem Hebepunkt über die Schultern von einem Verwender und hinunter auf einen oder mehrere Punkte an dem Torso des Verwenders, wobei das Lastübertragungsmittel (LMT) ein Gurtpaar (S1, S2; für Englisch: strap) umfasst, dass sich von dem Hebepunkt über entsprechende Schultern des Verwenders und hinunter zu dem einen oder mehreren Punkten an dem Torso des Verwenders erstreckt;
ii) entweder passives oder aktives Erzwingen von entweder einem oder beiden von einer angemessenen Rückenhaltung und einer Abfolge von Rückenhaltungen mit einem Haltungserfüllungsmittel (PCM; für Englisch: postural compliance means);
iii) Verbinden von dem Gurtpaar (S1, S2) des Lastübertragungsmittels (LTM) über ein Verbindungsmittel (C; für Englisch: coupling means), so dass das Gurtpaar (S1, S2) eine Kraft auf das Haltungserfüllungsmittel (PCM) in einer Richtung ausübt, die nicht zu einer Ausdehnungsrichtung von der Wirbelsäule des Trägers parallel ist, wobei das Verbindungsmittel (C) so konfiguriert ist, dass es benachbart zur Wirbelsäule des Trägers positioniert ist, wobei jeder Gurt (S1, S2) des Lastübertragungsmittels (LTM) mit dem entsprechenden Punkt des Haltungserfüllungsmittels (PCM) über das Verbindungsmittel (C) verbunden ist; und
iv) Verschieben von dem Verbindungsmittel (C) entlang eines Kanals, der sich in der Ausdehnungsrichtung von der Wirbelsäule des Trägers erstreckt, wobei der Kanal durch einen kannelierten Schieber (SL; für Englisch: slider) definiert wird, der benachbart zur Wirbelsäule des Trägers an dem Haltungserfüllungsmittel (PCM) positioniert ist.

## Revendications

1. Dispositif d'assistance au levage comprenant :
i) un moyen de transfert de charge (LTM), comprenant une paire de sangles (S1, S2), chaque sangle (S1, S2) étant configurée pour s'étendre depuis un point de levage par-dessus une épaule respective du porteur et vers le bas jusqu'à un ou plusieurs points sur le torse ;
ii) un moyen de conformité posturale (PCM), pour renforcer passivement ou activement la posture de dos appropriée et/ou une séquence de postures de dos ; et,
iii)un moyen de couplage (C), pour coupler la paire de sangles (S1, S2) du moyen de transfert de charge (LTM) à des points respectifs du moyen de conformité posturale (PCM) de telle sorte que la paire de sangles (S1, S2) exerce une force sur le moyen de conformité posturale (PCM) dans une direction qui n'est pas parallèle à une direction d'extension de la colonne vertébrale du porteur, le moyen de couplage (C) étant configuré pour être positionné adjacent à la colonne vertébrale du porteur, chaque sangle (S1, S2) du moyen de transfert de charge (LTM) étant couplée au point respectif du moyen de conformité posturale (PCM) par l'intermédiaire du moyen de couplage (C),
**caractérisé en ce qu'**un canal de coulissement est configuré pour être positionné adjacent à la colonne vertébrale du porteur sur le moyen de conformité posturale (PCM), dans lequel le moyen de couplage peut coulisser le long d'un canal défini par le canal de coulissement (SL) et s'étendant dans la direction d'extension de la colonne vertébrale du porteur.

2. Dispositif d'assistance au levage selon la revendication 1, dans lequel la posture de dos appropriée pour chaque niveau d'engagement du moyen de conformité posturale (PCM) est une posture qui favorise le maintien d'une courbure naturelle du dos de l'utilisateur à ce niveau d'engagement du moyen de conformité posturale (PCM).

3. Dispositif d'assistance au levage selon la revendication 1, dans lequel la posture de dos appropriée pour chaque niveau d'engagement du moyen de conformité posturale (PCM) est une posture qui réduit la flexion lombaire de pic à ce niveau d'engagement du moyen de conformité posturale (PCM).

4. Dispositif d'assistance au levage selon la revendication 1, comprenant en outre un moyen de couplage de levage pour chacun des bras de l'utilisateur, dans lequel chaque moyen de couplage de levage transfère pour ce bras au moyen de transfert de charge (LTM) au moins une partie du poids de la charge à soulever.

5. Dispositif d'assistance au levage selon la revendication 4, dans lequel le moyen de couplage de levage est choisi à partir du groupe consistant dans des gants, des crochets, des préhenseurs et des sangles de préhension, et des attaches à crochet et boucle.

6. Dispositif d'assistance au levage selon la revendication 4, dans lequel le moyen de couplage de levage est au moins un gant comprenant une pluralité d'éléments linéaires s'étendant depuis les extrémités des doigts du gant jusqu'à un point central adjacent à une portion de poignet du gant, ledit point central étant adapté pour être couplé au moyen de transfert de charge (LTM).

7. Dispositif d'assistance au levage selon la revendication 1, dans lequel un engagement croissant du moyen de conformité posturale est en relation linéaire avec le poids supporté par le moyen de transfert de charge (LTM).

8. Dispositif d'assistance au levage selon la revendication 1, dans lequel l'engagement croissant du moyen de conformité posturale (PCM) est en relation non linéaire avec le poids supporté par le moyen de transfert de charge (LTM).

9. Dispositif d'assistance au levage selon la revendication 8, dans lequel l'engagement croissant du moyen de conformité posturale (PCM) et un engagement à deux états allant d'un état dégagé à un état de plein engagement.

10. Dispositif d'assistance au levage selon la revendication 1, comprenant en outre un ou plusieurs capteurs pour effectuer une ou plusieurs mesures de la charge à soulever, en prise sur un ou plusieurs points sur le corps de l'utilisateur, ou un ou plusieurs indicateurs des contraintes provoquées par le levage sur le corps de l'utilisateur.

11. Dispositif d'assistance au levage selon la revendication 10, dans lequel un ou plusieurs capteurs comprennent un ou plusieurs capteurs de charge dangereuse.

12. Procédé pour réduire les blessures causées par le levage, comprenant le levage en portant un dispositif d'assistance au levage, comprenant :
i) transférer un poids d'une charge depuis un point de levage par-dessus les épaules d'un utilisateur et vers le bas jusqu'à un ou plusieurs points du torse de l'utilisateur par l'intermédiaire d'un moyen de transfert de charge (LTM) qui comprend une paire de sangles (S1, S2) qui s'étendent depuis le point de levage par-dessus les épaules respectives de l'utilisateur et vers le bas jusqu'à un ou plusieurs points sur le torse de l'utilisateur ;
ii) renforcer passivement ou activement l'une ou les deux d'une posture de dos appropriée et d'une séquence de postures de dos avec un moyen de conformité posturale (PCM),
iii)coupler sur la paire de sangles (S1, S2) du moyen de transfert de charge (LTM) par l'intermédiaire d'un moyen de couplage (C) de telle sorte que la paire de sangles (S1, S2) exerce une force sur le moyen de conformité posturale (PCM) dans une direction qui n'est pas parallèle à une direction d'extension de la colonne vertébrale du porteur, le moyen de couplage (C) étant configuré pour être positionné adjacent à la colonne vertébrale du porteur, chaque sangle (S1, S2) du moyen de transfert de charge (LTM) étant couplée au point respectif du moyen de conformité posturale (PCM) par l'intermédiaire du moyen de couplage (C) ; et
iv)faire coulisser le moyen de couplage (C) le long d'un canal s'étendant dans la direction d'extension de la colonne vertébrale du porteur, le canal étant défini par un canal de coulissement (SL) positionné adjacent à la colonne vertébrale du porteur sur le moyen de conformité posturale (PCM).
